# FASCICULE DE BREVET EUROPEEN

(11) **EP 1 641 763 B1**
(45) Date de publication et mention de la délivrance du brevet: **02.09.2009**
(21) Numéro de dépôt: 04767436.1
(22) Date de dépôt: 24.06.2004
(51) Int. Cl.: C07D 231/14, C07D 401/06, A61K 31/415, A61K 31/4439, A61P 3/04, A61P 25/14, A61P 25/18, A61P 25/28, A61P 25/30, A61P 29/00, A61P 37/00

(54) **DERIVES DE 4-CYANOPYRAZOLE-3-CARBOXAMIDE, LEUR PREPARATION ET LEUR APPLICATION COMME ANTAGONISTES DES RECEPTEURS AUX CANNABINOIDES CB1**
4-CYANOPYRAZOL-3-CARBONSÄUREAMIDDERIVATE, HERSTELLUNG UND THERAPEUTISCHE ANWENDUNG DAVON
4-CYANOPYRAZOLE-3-CARBOXAMIDE DERIVATIVES PREPARATION AND THERAPEUTIC APPLICATION THEREOF

(30) Priorité: 25.06.2003 FR 0307698
(43) Date de publication de la demande: 05.04.2006
(73) Titulaire: Sanofi-Aventis, 75013 Paris (FR)
(72) Inventeur: BARTH, Francis, F-34680 Saint-Georges d'Orques (FR); CONGY, Christian, F-34980 Saint Gely du Fesc (FR); MARTINEZ, Serge, F-34000 Montpellier (FR); RINALDI-CARMONA, Murielle, F-34680 Saint Georges d'Orques (FR)
(74) Mandataire: Kugel, Dominique
(86) Numéro de dépôt international: PCT/FR2004/001580
(87) Numéro de publication internationale: WO 2005/000820

(56) Documents cités:
- WO-A-97/19063
- WO-A-98/32441
- MESCHLER, J. P. ET AL: "Inverse agonist properties of N-(piperidin-1-yl)-5-(4-chlorophenyl)-1-(2 , dichlorophenyl)-4-methyl-1H-pyrazole-3- carboxamide HCl (SR141716A) and 1-(2-chlorophenyl)-4-cyano-5-(4-methoxyphe nyl)-1H-pyrazole-3-carboxylic acid phenylamide (CP-272871) for the CB1 cannabinoid receptor" BIOCHEMICAL PHARMACOLOGY, vol. 60, no. 9, 2000, pages 1315-1323, XP002262541 cité dans la demande
- DATABASE CHEMCATS CHEMICAL ABSTRACTS SERVICE, COLUMBUS, OHIO, US; XP002262542 & "Interchim Intermediates" 9 juillet 2002 (2002-07-09), INTERCHIM 213 AVENUE KENNEDY BP 1140 MONTLUCON, CEDEX, 03103, FRANCE
- SHAWALI, AHMAD SAMI: "Synthesis and tautomeric structure of some 2H-pyrazolo[3,4-d]pyridazines" JOURNAL OF HETEROCYCLIC CHEMISTRY, vol. 14, no. 3, 1977, pages 375-381, XP009021278 cité dans la demande

## Description

La présente invention se rapporte à des dérivés de 4-cyanopyrazole-3-carboxamide, leur préparation, leur application en thérapeutique.

Un dérivé de 4-cyanopyrazole-3-carboxamide est connu : le N-phényl-1-(2-chlorophényl)-4-cyano-5-(4-méthoxyphényl)-1*H*-pyrazole-3-carboxamide qui est décrit dans Biochem. Pharmacol., 2000, 60(9), 1315-1323, il est présenté comme ayant des propriétés antagonistes des récepteurs aux cannabinoïdes CB₁ et plus précisément des propriétés agonistes inverses desdits récepteurs.

La présente invention a pour objet un composé répondant à la formule (I) : 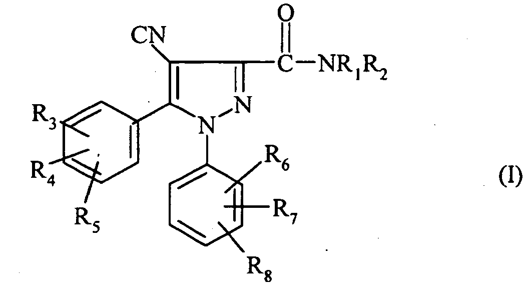 dans laquelle :
- R₁ représente l'hydrogène;
- R₂ représente un groupe choisi parmi le t-butyle et le 1,1-diméthylpropyle;
- R₃, R₄, R₅, R₆, R₇, R₈ représentent chacun indépendamment l'un de l'autre un atome d'hydrogène ou d'halogène, un groupe (C₁-C₆)alkyle, (C₁-C₆)alcoxy ou trifluorométhyle ; à la condition qu'au moins l'un des substituants R₃, R₄, R₅, R₆, R₇, R₈ soit différent de l'hydrogène ;
ainsi que leurs sels, leurs solvats et leurs hydrates.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de sel. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- groupe alkyle : un radical linéaire ou ramifié tel que méthyle, éthyle, propyle, isopropyle, butyle, isobutyle, *tert*-butyle, n-pentyle, isopentyle, n-hexyle, isohexyle, le groupe méthyle étant préféré pour un (C₁-C₄)alkyle ; les groupes *tert-*butyle, 1,1-diméthylpropyle et 2-méthylbutyl-2 étant préférés pour un (C₃-C₇)alkyle;
- groupe (C₁-C₄)alcoxy : un radical linéaire ou ramifié contenant 1 à 4 atomes de carbone, le groupe méthoxy étant préféré ;
- atome d'halogène : un atome de fluor, de chlore, de brome ou d'iode, les atomes de fluor, chlore ou brome étant préférés.

Les radicaux carbocycliques ou aromatiques en C₃-C₁₀ comprennent les radicaux mono ou polycycliques, condensés ou pontés. Les radicaux monocycliques incluent les cycloalkyles par exemple cyclopropyle, cyclobutyle, cyclopentyle, cyclohexyle, cycloheptyle, cyclooctyle ; le cyclohexyle et le cyclopentyle étant préférés. Les radicaux di- ou tri- cycliques condensés, pontés ou spiraniques incluent par exemple les radicaux bicyclo[2.2.1]heptanyle, bicyclo[3.1.1]heptanyle, bicyclo[2.2.2]octanyle, bicyclo[3.2.1]octanyle, bicyclo[3.2.1]octanyle, l'adamantyle, le bicyclo[3.1.1]heptanyle et le bicyclo[3.2.1]octanyle étant préférés.

Par radical hétérocyclique, saturé ou insaturé, de 5 à 10 atomes, contenant ou non un deuxième hétéroatome tel que O ou N, on entend des radicaux tel que morpholin-4-yle, pipéridin-1-yle, pipérazin-1-yle, pyrrolidin-1-yle, 3,6-dihydropyridin-1-yle, octahydrocyclopenta[c]pyrrol-2-yle, les radicaux pipéridin-1-yle et morpholin-4-yle étant préférés.

Selon la présente invention, on préfère tout particulièrement le 5-(4-bromophényl)-4-cyano-1-(2,4-dichlorophényl)-N-*tert*-butyl-1*H*-pyrazole-3-carboxamide, le 5-(4-bromophényl)-4-cyano-1-(2,4-dichlorophényl)-N-(1,1-diméthylpropyl)-1*H*-pyrazole-3-carboxamide, le 5-(4-chlorophényl)-4-cyano-1-(2,4-dichlorophényl)-N-pipéridin-1-yl-1*H*-pyrazole-3-carboxamide, le 5-(4-méthoxy phényl)-4-cyano-1-1(2,4-dichlorophényl)-N-*tert*-butyl-1*H*-pyrazole-3-carboxamide.

La présente invention a également pour objet un procédé de préparation des composés selon l'invention.

Ce procédé est **caractérisé en ce que** l'on traite un dérivé fonctionnel de l'acide 4-cyano-1,5-diphénylpyrazole-3-carboxylique de formule : 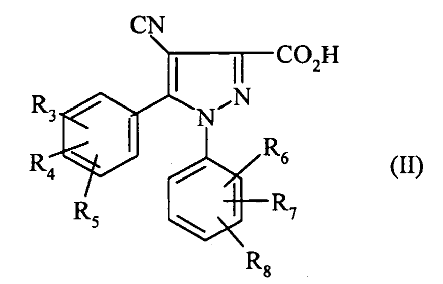 dans laquelle R₃, R₄, R₅, R₆, R₇, R₈ sont tels que définis pour (I) avec une amine de formule HNR₁R₂ (III) dans laquelle R₁ et R₂ sont tels que définis pour (I)
Eventuellement, on transforme le composé ainsi obtenu en un de ses sels ou solvats.

Comme dérivé fonctionnel de l'acide (II) on peut utiliser le chlorure d'un acide, l'anhydride, un anhydride mixte, un ester alkylique en C₁-C₄ dans lequel l'alkyle est droit ou ramifié, un ester activé, par exemple l'ester de *p*-nitrophényle, ou l'acide libre opportunément activé, par exemple, avec le N,N-dicyclohexylcarbodiimide ou avec l'hexafluorophosphate de benzotriazol-N-yloxotris(diméthylamino)phosphonium (BOP) ou hexafluorophosphate de benzotriazol-1-yloxotris(pyrrolidino)phosphonium (PyBOP).

Ainsi dans le procédé selon l'invention, on peut faire réagir le chlorure d'un acide pyrazole-3-carboxylique, obtenu par réaction du chlorure de thionyle sur l'acide de formule (II), avec une amine HNR₁R₂, dans un solvant inerte, tel qu'un solvant chloré (le dichlorométhane, le dichloroéthane, le chloroforme par exemple), un éther (tétrahydrofurane, dioxane par exemple), ou un amide (N,N-diméthylformamide par exemple) sous une atmosphère inerte, à une température comprise entre 0°C et la température ambiante, en présence d'une amine tertiaire telle que la triéthylamine, la N-méthylmorpholine ou la pyridine.

Une variante consiste à préprarer l'anhydride mixte de l'acide de formule (II) par réaction du chloroformiate d'éthyle avec l'acide de formule (II), en présence d'une base telle que la triéthylamine, et à le faire réagir avec une amine HNR₁R₂, dans un solvant tel que le dichlorométhane, sous une atmosphère inerte, à la température ambiante, en présence d'une base telle que la triéthylamine.

Les composés de formule (II) peuvent être préparés par différents procédés connus de la littérature par exemple tel que décrit dans J. Heterocyclic Chem., 1977, 14 (3), 375-381.

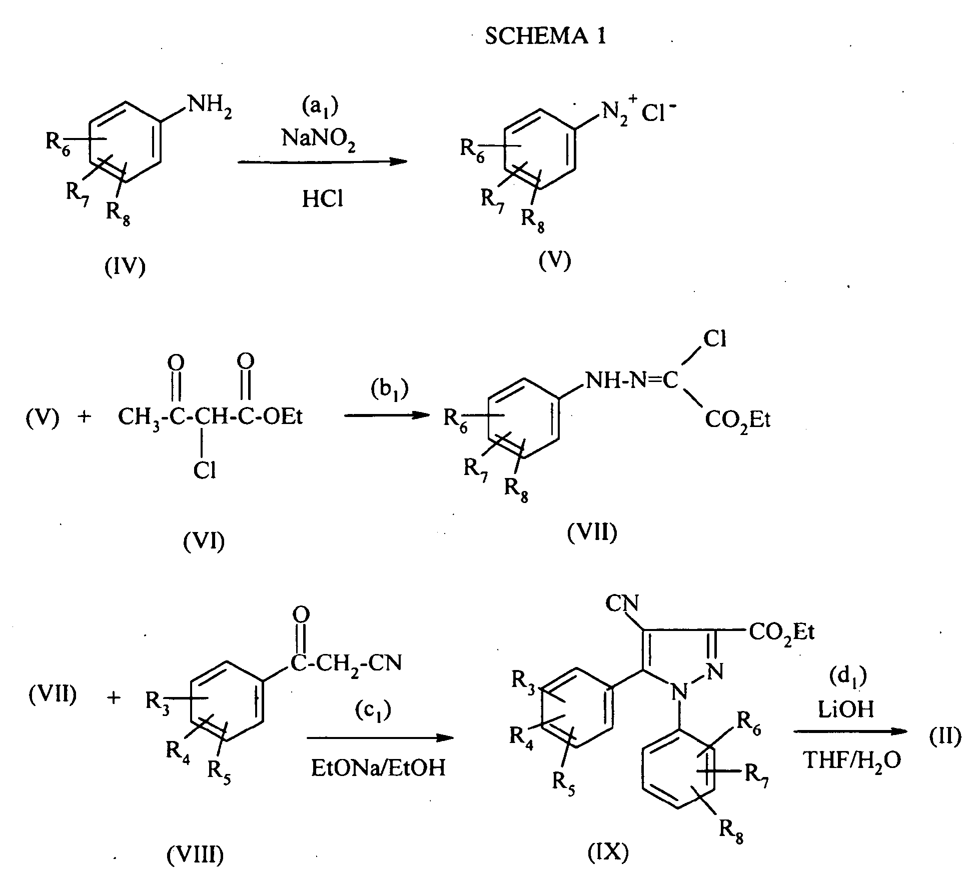

Dans l'étape a₁) une aniline de formule (IV) est transformée en sel de diazonium (V) par l'action d'un nitrite en milieu acide, comme décrit dans Razdan et al., Med. Chem. Res., 1995, 5, 54. Le sel de diazonium (V) est ensuite mis en réaction avec le 2-chloro-3-oxo-butanoate d'éthyle (VI) pour donner le dérivé d'hydrazone (VII).

Le dérivé d'hydrazone (VII) est condensé avec le nitrile de formule (VIII) en présence d'une base forte tel que l'éthylate de sodium dans l'éthanol pour obtenir le dérivé de pyrazole (IX). Celui-ci est enfin transformé en acide (II) par saponification en utilisant des conditions douces, par exemple LiOH dans un mélange THF/eau.

Les acides de formule (II) et leurs esters de formule (IX) sont généralement nouveaux. Certains de ces composés sont décrits dans J. Heterocyclic Chem., 1977, 14 (3), 375-381 ; l'ester éthylique de l'acide 1-(4-bromophényl)-4-cyano-5(4-méthoxyphényl)1*H*-pyrazole-3-carboxylique est cité dans le catalogue Interchim. Intermediates.

Ainsi la présente invention a également pour objet les composés de formule : 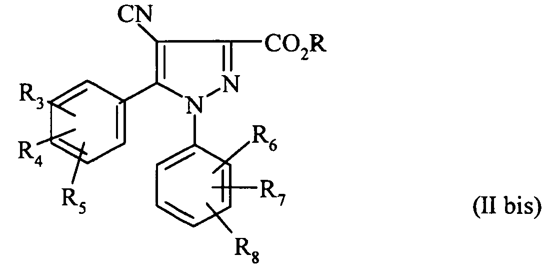 dans laquelle R est un atome d'hydrogène ou un groupe (C₁-C₄)alkyle et
- R₃ est en position 4- et représente un atome de chlore ou de brome ou un groupe méthyle, éthyle, triflorométhyle ou méthoxy ;
- R₆ est en position -2 et représente un atome de chlore;
- R₇ est en position -4 et représente un atome d'hydrogène ou un atome de chlore ;
- R₄, R₅ et R₈ représentent un atome d'hydrogène.

Plus particulièrement, on préfère les composés de formule (II bis) dans laquelle :
- R₃ est en position 4- et représente un atome de chlore ou de brome;
- R₆ et R₇ sont en position 2,4- et représentent deux atomes de chlore ;
- R₄, R₅ et R₈ représentent un atome d'hydrogène.

Les amines HNR₁R₂ sont connues ou préparées par des méthodes connues telles que celles décrites dans Chem. Ber., 1986, 119, 1413-1423.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Dans la présente description, on utilise les abréviations suivantes :
AcOEt : acétate d'éthyle
BOP : hexafluorophosphate de benzotriazolyloxy trisdiméthyl aminophosphonium
DCM : dichlorométhane
DMF : diméthylformamide
EtOH : éthanol
F : point de fusion
iPr₂O : éther isopropylique
TA : température ambiante
THF : tétrahydrofurane

Les composés selon l'invention sont analysés par couplage LC/UV/MS (chromatographie liquide/détection UV/spectrométrie de masse). On mesure le pic moléculaire (MH⁺) et le temps de rétention (t) en minutes.

On utilise une colonne Xterra Waters^{®} MS C18, commercialisée par Waters, de 2,1 x 30 mm, 3,5 µm, à température ambiante, débit 1 mL/minute.

L'éluant est composé comme suit :
- solvant A : 0,025 % d'acide trifluoroacétique (TFA) dans l'eau
- solvant B : 0,025 % de TFA dans l'acétonitrile.

Gradient : Le pourcentage de solvant B varie de 0 à 100 % en 2 minutes avec un plateau à 100 % de B pendant 1 minute.

La détection UV est effectuée entre 210 nm et 400 nm et la détection de masse en mode ionisation chimique à pression atmosphérique.

Les spectres de RMN onst enregistrés à 200 MHz dans le DMSO-d₆.

Pour l'interprétation des spectres de résonance magnétique nucléaire (RMN) les abréviations suivantes sont utilisées : s : singulet ; d : doublet ; m : massif : se : singulet élargi ; dd : doublet de doublet ; mt multiplet.

### Préparation 1

### Acide 5-(4-chlorophényl)-4-cyano-1-(2,4-dichlorophényl)-1H-pyrazole-3-carboxylique.

### A) Chloro[(2,4-dichlorophényl)hydrazono]acétate d'éthyle.

On mélange sous agitation 7,3 g de dichloroaniline dans 75 ml d'une solution d'HCl à 24 % et 200 ml d'eau et on maintient l'agitation pendant 2 heures. On refroidit au bain de glace et on ajoute goutte à goutte en 30 minutes une solution contenant 3,1 g de NaNO₂ dans 21 ml d'eau. Le mélange obtenu est ajouté à une solution contenant 3,51 g d'acétate de sodium et 6,21 ml de 2-chloro-3-oxobutanoate d'éthyle dans 450ml d'EtOH, refroidie au bain de glace. On laisse remonter lentenement la température en maintenant l'agitation. Le précipité formé est filtré, lavé à l'eau puis séché sous vide. On obtient 11,43 g du composé attendu.

RMN : 1,40 ppm : t : 3H ; 4,40 ppm : d : 2H ; 7,40-7,80 ppm : m : 3H ; 9,25 ppm : s : 1H.

### B) 5-(4-chlorophényl)-4-cyano-1-(2,4-dichlorophényl)-1H-pyrazole-3-carboxylate d'éthyle.

On chauffe à reflux pendant 18 heures un mélange contenant 3,27 g du composé de l'étape précédente, 1,99 g de 3-(4-chlorophényl)-3-oxopropanenitrile dans 120 ml d'EtOH et de l'éthylate de sodium préparé en mélangeant 0,28 g de sodium dans 25 ml d'EtOH. Après retour à TA, on évapore à sec puis on reprend dans 150 ml d'AcOEt, filtre le précipité formé et lave la phase organique par de l'eau puis par une solution saturée de NaCl. L'huile obtenue est chromatographiée sur silice en éluant par un mélange AcOEt/toluène (2/98 à 3/97 ; v/v). Le solide obtenu est recristallisé 2 fois dans un mélange CH₂Cl₂/iPr₂O pour donner 1,12 g du composé attendu sous forme de cristaux blancs, F = 112°C.

### C) Acide 5-(4-chlorophényl)-4-cyano-1-(2,4-dichlorophényl)-1H-pyrazole-3-carboxylique

On mélange 0,436 g d'ester obtenu à l'étape précédente dans 25 ml de THF et 50 mg de LiOH dans 5 ml d'eau et on chauffe 2 heures à 65°C. On concentre de moitié, on verse le milieu réactionnel dans 50ml d'eau glacée et 5 ml d'HCl à 5%. Le mélange organique est extrait par CH₂Cl₂ puis lavé par NaCl. On obtient 0,41 g du composé attendu sous forme solide.F = 132-137°C.

RMN: 7,42 ppm : d : 2H ; 7,61 ppm : d : 2H ; 7,69 ppm : dd : 1H ; 7,89 ppm : s : 1H ; 7,92 ppm : d : 1H ; 13,8-14,6 ppm : se : 1H.

### Préparation 2

### Acide 5-(4-bromophényl)-4-cyano-1-(2,4-dichlorophényl)-1H-pyrazole-3-carboxylique

### A) 3-(4-bromophényl)-3-oxopropanenitrile

On prépare une solution contenant 9,4 g de KCN dans 20 ml d'eau puis on la verse goutte à goutte sur un mélange contenant, 20 g de 2-bromo-1-(4-bromophényl)éthanone dissous dans 800 ml d'éthanol à 90 %. Après 5 heures sous agitation à TA, on filtre le solide formé puis on le rince à l'eau glacée. Le solide obtenu est dissous dans 400ml d'eau puis on ajoute du charbon actif, on laisse 20 minutes sous agitation, puis on filtre sur Célite^{®}. Le filtrat obtenu est traité par HCl à 10 % et le précipité blanc formé est filtré, lavé à l'eau puis séché sous vide. On obtient 7,63 g du composé attendu, F = 164°C.

RMN : 4,75 ppm : se : 2H ; 7,80 ppm : mt : 4H.

### B) 5-(4-bromophényl)-4-cyano-1-(2,4-dichlorophényl)-1H-pyrazole-3-carboxylate d'éthyle

On prépare une solution d'éthylate de sodium en mélangeant 0,86 g de sodium dans 107 ml d'EtOH, on ajoute rapidement 7,63 g du composé préparé à l'étape précédente dans 610 ml d'EtOH puis 9,2 g du composé obtenu à l'étape A de la préparation 1 et on laisse le milieu réactionnel une nuit sous agitation à TA. On filtre l'insoluble puis on concentre le filtrat sous vide. Le produit obtenu est concentré sur silice en éluant par un mélange toluène/AcOEt (96/4 ; v/v). On obtient 5 g du composé attendu.

RMN : 1,25 ppm : t : 3H ; 4,30 ppm : q : 2H ; 7,20 ppm : d : 2H ; 7,50-7,70 ppm : m : 3H ; 7,70-8,00 ppm : m : 2H.

### C) Acide 5-(4-bromophényl)-4-cyano-1-(2,4-dichlorophényl)-1H-pyrazole-3-carboxylique

On place 2,8 g d'ester obtenu à l'étape précédente dans 90 ml de THF et on ajoute 0,4 g de LiOH dans 8 ml d'eau puis on chauffe à 65°C pendant 3 heures. On verse le milieu réactionnel sur un mélange de 240 ml d'eau glacée et 16 ml d'HCl à 10 %. La phase organique est extraite par CH₂Cl₂ puis lavée par une solution saturée de NaCl. On obtient 2,3 g du composé attendu.

RMN : 7,30 ppm : d : 2H ; 7,60-7,80 ppm : m : 3H ; 7,80-8,00 ppm : m : 2H ; 14,15 ppm : se : 1H.

En espérant selon les modes opératoires des préparations ci-dessus, on a obtenu les composés du tableau suivant :

**TABLEAU 1**

| | | | |
|---|---|---|---|
| 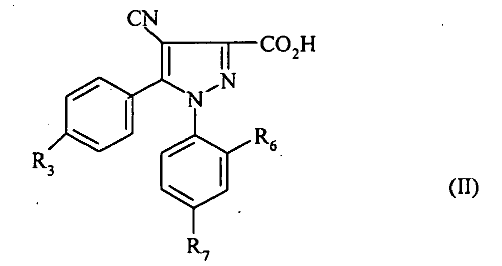 | | | |

| Préparation | R₃ | R₆, R₇ | Point de fusion |
|---|---|---|---|
| 3 | -OMe | -Cl, -Cl | F = 230°C |
| 4 | -Me | -Cl, -Cl | F = 223°C |
| 5 | -CF₃ | -Cl, -Cl | F = 238°C |
| 6 | -Et | -Cl, H | F = 254°C |

### EXEMPLE 1 : composé 1

### 5-(4-Chlorophényl)-4-cyano-1-(2,4-dichlorophényl)-N-pipéridin-1-yl-1H-pyrazole-3-carboxamide.

On ajoute goutte à goutte 0,39 g de l'acide de la Préparation 1 à une solution contenant 0,20 ml de 1-aminopipéridine et 0,50 ml de triéthylamine dans 15 ml de CH₂Cl₂ puis on ajoute goutte à goutte 0,80 g de BOP et on laisse sous agitation à TA pendant 20 heures. Le milieu réactionnel est hydrolysé à l'eau puis on lave la phase organique par une solution d'HCl à 2 %, par une solution de Na₂CO₃ à 5% puis par une solution saturée de NaCl. Après séchage, le produit obtenu est chromatographié sur silice en éluant par un mélange MeOH/CH₂Cl₂ (0,5/99,5 ; v/v) pour donner une mousse. Le composé attendu cristallise dans un mélange CH₂Cl₂/iPr₂O, on obtient 0,28 g, F = 227-229°C.

### EXEMPLE 2 : composé 12

### 5-(4-Bromophényl)-4-cyano-1-(2,4-dichlorophényl)-N-tert-butyl-1H-pyrazole-3-carboxamide.

Dans 20 ml de CH₂Cl₂, on mélange 0,437g de l'acide de la Préparation 2, 0,19 ml de *tert*-butylamine, 0,5 ml de NEt₃, puis 0,79 g de BOP et on laisse sous agitation à TA pendant 48 heures. Le milieu réactionnel est hydrolysé à l'eau puis la phase organique est lavée par une solution d'HCl à 2 %, une solution de Na₂CO₃ à 5 % puis une solution saturée de NaCl. Après séchage, le produit obtenu est chromatograpié sur silice en éluant par un mélange toluène/AcOEt (95/5 ; v/v) pour donner un produit qui cristallise dans l'éther isopropylique. On obtient 370 mg, F = 184°C.

RMN : 1,35 ppm : s : 9H ; 7,30 ppm : d : 2H ; 7,60-8,00 ppm : m : 6H.

Le tableau qui suit illustre les structure chimiques et les propriétés physiques de quelques exemples de composé selon l'invention.

Dans ce tableau Me, Et et tBu représentent respectivement les groupes méthyle, éthyle et *tert*-butyle.

**TABLEAU 2**

| | | | | |
|---|---|---|---|---|
| 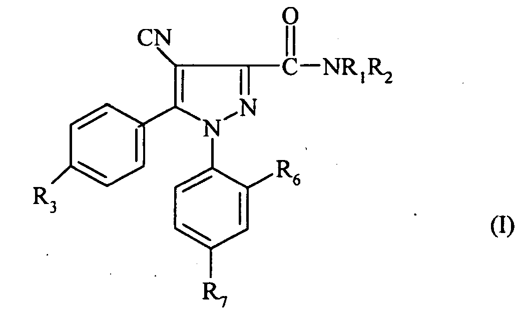 | | | | |

| Composés | R₃ | R₆,R₇ | -NR₁R₂ | Caractérisation |
|---|---|---|---|---|
| 3 | -Cl | -Cl, -Cl | -NH-tBu | F = 197°C |
| 4 | -Cl | -Cl, -Cl | 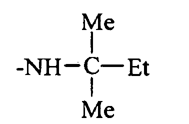 | F = 159°C |
| 11 | -Br | -Cl, -Cl | 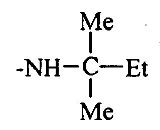 | F = 160°C |
| 12 | -Br | -Cl, -Cl | -NH-tBu | F = 184°C |
| 23 | -OMe | -Cl, -Cl | -NH-tBu | F = 229°C |
| 25 | -Et | -Cl, -Cl | -NH-tBu | F = 225°C |
| 26 | -Et | -Cl, H | 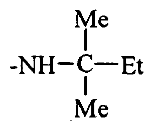 | F = 192°C |
| 28 | -CF₃ | -Cl, -Cl | -NH-tBu | F = 192°C |
| 30 | -CF₃ | -Cl, -Cl | 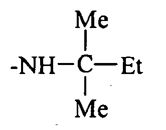 | F = 177°C |
| 31 | -OMe | -Cl, -Cl | -NH-tBu | F = 157°C |
| 33 | -OMe | -Cl, -Cl | 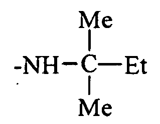 | F = 143°C |
| 34 | -Me | -Cl, -Cl | -NH-tBu | F = 171°C |
| 36 | -Me | -Cl, -Cl | 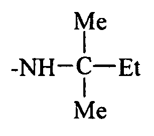 | F = 157°C |

Les composés selon l'invention ont fait l'objet d'essais pharmacologiques permettant de déterminer leur effet antagoniste des récepteurs aux cannabinoïdes CB ₁.

Les composés de formule (I) possèdent une très bonne affinité *in vitro* (IC₅₀ ≤ 10⁻⁷M) pour les récepteurs aux cannabinoïdes CB₁, dans les conditions expérimentales décrites par M. Rinaldi-Carmona et al. (FEBS Letters, 1994, 350, 240-244).

La nature antagoniste des composés de formule (I) a été démontrée par les résultats obtenus dans les modèles de l'inhibition de l'adénylate-cyclase comme décrits dans M. Rinaldi-Carmona et al., J. Pharmacol. Exp. Ther., 1996, 278, 871-878 et M. Bouaboula et al., J. Biol. Chem., 1997, 272, 22330-22339.

Les composés selon l'invention ont été testés *in vivo* (binding *ex vivo*) chez la souris après l'administration intraveineuse et/ou orale, selon les conditions expérimentales décrites par Rinaldi-Carmona et al. (J. Pharmacol. Exp., 1998, 284, 644-650). Par voie intraveineuse, la dose efficace (DE₅₀) de ces composés pour les récepteurs CB ₁ est inférieure ou égale à 10 mg/kg. Par voie orale, les composés 2, 3, 4, 11 et 12 ont une DE₅₀ comprise entre 1 et 20 mg/kg pour les récepteurs CB₁.

La toxicité des composés de formule (I) est compatible avec leur utilisation en tant que médicament.

Selon un autre de ses aspects, la présente invention concerne l'utilisation d'un composé de formule (I), ou de l'un de ses sels, solvats ou hydrates pharmaceutiquement acceptables, pour la préparation de médicaments destinés à traiter ou à prévenir les maladies impliquant les récepteurs aux cannabinoïdes CB₁.

Par exemple et de manière non limitative, les composés de formule (I) sont utiles comme médicaments psychotropes, notamment pour le traitement des désordres psychiatriques incluant l'anxiété, la dépression, les troubles de l'humeur, l'insomnie, les troubles délirants, les troubles obsessionnels, les psychoses en général, la schizophrénie, les troubles de l'attention et de l'hyperactivité (TDAH), notamment chez les enfants hyperkinétiques (MBD), ainsi que pour le traitement des troubles liés à l'utilisation de substances psychotropes, notamment dans le cas d'un abus d'une substance et/ou de dépendance à une substance, y compris la dépendance alcoolique et la dépendance nicotinique.

Les composés de formule (I) selon l'invention peuvent être utilisés comme médicaments pour le traitement de la migraine, du stress, des maladies d'origine psychosomatique, des crises d'attaques de panique, de l'épilepsie, des troubles du mouvement, en particulier des dyskinésies ou de la maladie de Parkinson, des tremblements et de la dystonie.

Les composés de formule (I) selon l'invention peuvent également être utilisés comme médicaments dans le traitement des troubles mnésiques, des troubles cognitifs, en particulier dans le traitement des démences séniles, de la maladie d'Alzheimer, ainsi que dans le traitement des troubles de l'attention ou de la vigilance. De plus, les composés de formule (I) peuvent être utiles comme neuroprotecteurs, dans le traitement de l'ischémie, des traumatismes crâniens et le traitement des maladies neurodégénératives : incluant la chorée, la chorée de Huntington, le syndrome de Tourrette.

Les composés de formule (I) selon l'invention peuvent être utilisés comme médicaments dans le traitement de la douleur : les douleurs neuropathiques, les douleurs aiguës périphériques, les douleurs chroniques d'origine inflammatoire.

Les composés de formule (I) selon l'invention peuvent être utilisés comme médicaments dans le traitement des troubles de l'appétit, de l'appétence (pour les sucres, carbohydrates, drogues, alcools ou toute substance appétissante) et/ou des conduites alimentaires, notamment en tant qu'anorexigènes ou pour le traitement de l'obésité ou de la boulimie ainsi que pour le traitement du diabète de type II ou diabète non insulinodépendant et pour le traitement des dyslipidémies, du syndrome métabolique. De plus, les composés de formule (I) selon l'invention peuvent être utilisés en tant que médicaments dans le traitement des troubles gastro-intestinaux, des troubles diarrhéiques, des ulcères, des vomissements, des troubles vésicaux et urinaires, des troubles d'origine endocrinienne, des troubles cardio-vasculaires, de l'hypotension, du choc hémorragique, du choc septique, de la cirrhose chronique du foie, de l'asthme, de la bronchite chronique et de la broncho-pneumopathie chronique obstructive, du syndrome de Raynaud, du glaucome, des troubles de la fertilité, des phénomènes inflammatoires, des maladies du système immunitaire, en particulier autoimmunes et neuroinflammatoires tel que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux ainsi qu'en tant que médicaments pour la chimiothérapie anticancéreuse et pour le traitement du syndrome de Guillain-Barré.

Selon la présente invention, les composés de formule (I) sont tout particulièrement utiles pour le traitement des troubles psychotiques, en particulier la schizophrénie, les troubles de l'attention et de l'hyperactivité (TDAH), notamment chez les enfants hyperkinétiques (MBD) ; pour le traitement des troubles de l'appétit et de l'obésité pour le traitement des troubles mnésiques et cognitifs ; pour le traitement de la dépendance alcoolique, de la dépendance nicotinique, c'est à dire pour le sevrage alcoolique et pour le sevrage tabagique ; et pour le traitement des dyslipidémies, du syndrome métabolique.

Selon un de ses aspects, la présente invention est relative à l'utilisation d'un composé de formule (I) de ses sels pharmaceutiquement acceptables et de leurs solvats ou hydrates pour le traitement des troubles et maladies indiqués ci-dessus.

Les composés de formule (I) selon l'invention peuvent être utilisés en association avec un ou plusieurs autres principes actifs utiles pour la prévention et/ou le traitement des maladies indiquées ci-dessus : à titre d'exemple de principes actifs pouvant être associés à un composé de formule (I), on peut citer les antipsychotiques, les anxiolytiques, des agents améliorant la mémoire, des agents anti-parkinson, des anti-épileptiques, des anorexigènes ou d'autres agents anti-obésité, des agonistes nicotiniques, des inhibiteurs de la monoamine oxidase, des analgésiques, des anti-inflammatoires, des antihypertenseurs tels que : antagonistes des récepteurs AT₁ de l'angiotensine II, inhibiteurs de l'enzyme de conversion, antagonistes calciques, beta-bloquants, des anti-diabétiques, des anti-hyperlipémiants, des anti-hypercholestérolémiants, des agonistes PPAR (de l'anglais peroxisome proliferator activated receptor).

Le composé selon l'invention est généralement administré en unité de dosage.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I), un de ses sels pharmaceutiquement acceptables ou un de leurs solvats.

Le composé de formule (I) ci-dessus et ses sels ou solvats pharmaceutiquement acceptables peuvent être utilisés à des doses journalières de 0,01 à 100 mg par kg de poids corporel du mammifère à traiter, de préférence à des doses journalières de 0,02 à 50 mg/kg. Chez l'être humain, la dose peut varier de préférence de 0,05 à 4000 mg par jour, plus particulièrement de 0.1 à 1000 mg par jour selon l'âge du sujet à traiter ou le type de traitement, à savoir prophylactique ou curatif. Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Dans les compositions pharmaceutiques de la présente invention pour l'administration orale, sublinguale, inhalée, sous-cutanée, intramusculaire, intraveineuse, transdermique, locale ou rectale, le principe actif peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intra-oculaire et les formes d'administration rectale.

Dans les compositions pharmaceutiques de la présente invention, le principe actif est généralement formulé en unités de dosage contenant de 0,05 à 1000 mg, avantageusement de 0,1 à 500 mg, de préférence de 1 à 200 mg dudit principe actif par unité de dosage pour les administrations quotidiennes.

A titre d'exemple, une forme unitaire d'administration d'un composé selon l'invention sous forme de comprimé peut comprendre les composants suivants :

| | |
|---|---|
| Composé selon l'invention : | 50,0 mg |
| Mannitol : | 223,75 mg |
| Croscarmellose sodique : | 6,0 mg |
| Amidon de maïs : | 15,0 mg |
| Hydroxypropyl-méthylcellulose : | 2,25 mg |
| Stéarate de magnésium : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé répondant à la formule (I) : 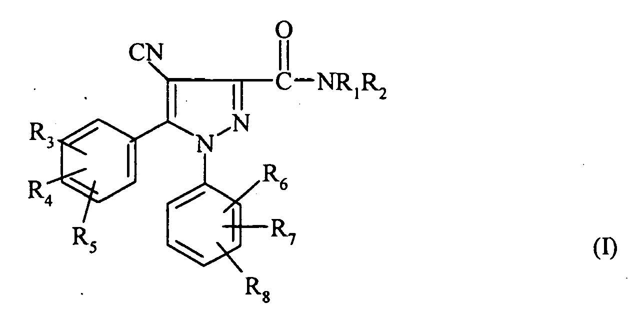 dans laquelle :
- R₁ représente l'hydrogène ;
- R₂ représente un groupe choisi parmi le t-butyle et le 1,1-diméthylpropyle ;
- R₃, R₄, R₅, R₆, R₇, R₈ représentent chacun indépendamment l'un de l'autre un atome d'hydrogène ou d'halogène, un groupe (C₁-C₆)alkyle, (C₁-C₆)alcoxy ou trifluorométhyle ; à la condition qu'au moins l'un des substituants R₃, R₄, R₅, R₆, R₇, R₈ soit différent de l'hydrogène ;
à l'état de base ou de sel d'addition à un acide, ainsi qu'à l'état d'hydrate ou de solvat.

2. Composé selon la revendication 1 choisi parmi le 5-(4-bromophényl)-4-cyano-1-(2,4-dichlorophényl)-N-*tert*-butyl-1*H*-pyrazole-3-carboxamide et le 5-(4-bromophényl)-4-cyano-1-(2,4-dichlorophényl)-N-(1,1-diméthylpropyl)-1*H-*pyrazole-3-carboxamide ;
à l'état de base ou de sel d'addition à un acide, ainsi qu'à l'état d'hydrate ou de solvat.

3. Procédé de préparation d'un composé de formule (I) selon la revendication 1 **caractérisé en ce que** l'on traite un dérivé fonctionnel de l'acide 4-cyano-1,5-diphénylpyrazole-3-carboxylique de formule : 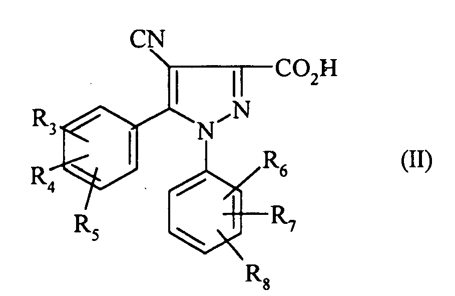 dans laquelle R₃, R₄, R₅, R₆, R₇ et R₈ sont tels que définis pour (I) à la revendication 1 avec une amine de formule HNR₁R₂ (III) dans laquelle R₁ et R₂ sont tels que définis pour (I) à la revendication 1.

4. Composé de formule : 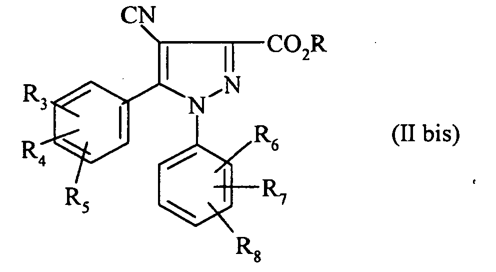 dans laquelle :
- R est un atome d'hydrogène ou un groupe (C₁-C₄)akyle ;
- R₃ est en position 4- et représente un atome de chlore ou de brome ou un groupe méthyle, éthyle, méthoxy ou trifluorométhyle ;
- R₆ est en position 2- et représente un atome de chlore ;
- R₇ est en position 4- et représente un atome d'hydrogène ou de chlore ;
- R₄, R₅ et R₈ représentent un atome d'hydrogène.

5. Médicament **caractérisé en ce qu'**il comprend un composé de formule (I) selon l'une quelconque des revendications 1 à 2, ou un sel d'addition de ce composé à un acide pharmaceutiquement acceptable, ou encore un hydrate ou un solvat.

6. Composition pharmaceutique **caractérisé en ce qu'**elle comprend un composé de formule (I) selon l'une quelconque des revendications 1 à 2 ou un sel pharmaceutiquement acceptable, un hydrate ou un solvat de ce composé ainsi qu'au moins un excipient pharmaceutiquement acceptable.

7. Utilisation d'un composé de formule (I) selon l'une quelconque des revendications 1 à 2 pour la préparation d'un médicament destiné au traitement des troubles de l'appétit, des troubles gastro-intestinaux, des phénomènes inflammatoires, des maladies du système immunitaire, des troubles psychotiques, de la dépendance alcoolique, de la dépendance nicotinique.

8. Composé de formule (I) selon l'une quelconque des revendications 1 à 2 pour utilisation pour le traitement des troubles de l'appétit, des troubles gastro-intestinaux, des phénomènes inflammatoires, des maladies du système immunitaire, des troubles psychotiques, de la dépendance alcoolique, de la dépendance nicotinique.

## Claims

1. Compound corresponding to formula (I): 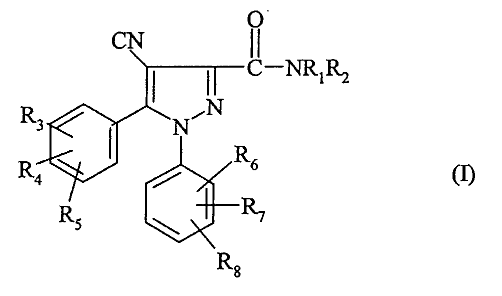 in which:
- R₁ represents hydrogen;
- R₂ represents a group chosen from t-butyl and 1,1-dimethylpropyl;
- R₃, R₄, R₅, R₆, R₇, R₈ represent, each independently of the other, a hydrogen or halogen atom, a (C₁-C₆)-alkyl, (C₁-C₆)alkoxy or trifluoromethyl group; provided that at least one of the substituents R₃, R₄, R₅, R₆, R₇, R₈ is different from hydrogen;
in the form of a base or an addition salt with an acid, and in the form of a hydrate or a solvate.

2. Compound according to Claim 1, chosen from 5-(4-bromophenyl)-4-cyano-1-(2,4-dichlorophenyl)-N-*tert*-butyl-1*H*-pyrazole-3-carboxamide and 5-(4-bromophenyl)-4-cyano-1-(2,4-dichlorophenyl)-N-(1,1-dimethylpropyl)-1*H*-pyrazole-3-carboxamide;
in the form of a base or an addition salt with an acid, and in the form of a hydrate or a solvate.

3. Method for preparing a compound of formula (I) according to Claim 1, **characterized in that** a functional derivative of 4-cyano-1,5-diphenylpyrazole-3-carboxylic acid of formula: 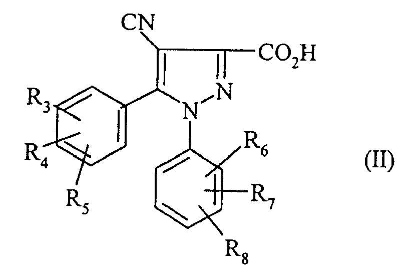 in which R₃, R₄, R₅, R₆, R₇, R₈ are as defined for (I) in claim 1, is treated with an amine of formula HNR₁R₂ (III) in which R₁ and R₂ are as defined for (I) in Claim 1.

4. Compound of formula: 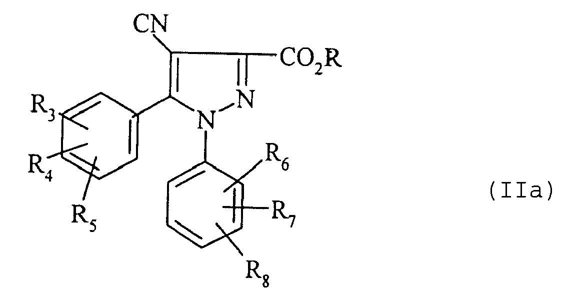 in which:
- R is a hydrogen atom or a (C₁-C₄) alkyl group;
- R₃ is at the 4-position and represents a chlorine or bromine atom or a methyl, ethyl, methoxy or trifluoromethyl group;
- R₆ is at the 2-position and represents a chlorine atom;
- R₇ is at the 4-position and represents a hydrogen or chlorine atom;
- R₄, R₅ and R₈ represent a hydrogen atom.

5. Medicament, **characterized in that** it comprises a compound of formula (I) according to either one of Claims 1 and 2, or an addition salt of this compound with a pharmaceutically acceptable acid, or a hydrate or a solvate.

6. Pharmaceutical composition, **characterized in that** it comprises a compound of formula (I) according to either one of Claims 1 and 2 or a pharmaceutically acceptable salt, a hydrate or a solvate of this compound, and at least one pharmaceutically acceptable excipient.

7. Use of a compound of formula (I) according to either one of Claims 1 and 2 for the preparation of a medicament intended for the treatment of appetite disorders, gastrointestinal disorders, inflammatory phenomena, immune system diseases, psychotic disorders, alcohol dependence and nicotine dependence.

8. Compound of formula (I) according to either one of Claims 1 and 2 for use in the treatment of appetite disorders, gastrointestinal disorders, inflammatory phenomena, immune system diseases, psychotic disorders, alcohol dependence and nicotine dependence.

## Patentansprüche

1. Verbindung der Formel (I): 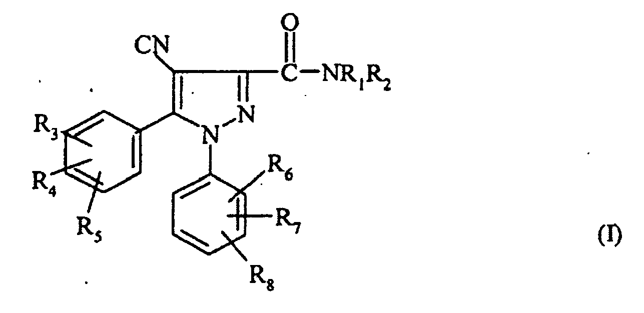 worin:
- R¹ für Wasserstoff steht;
- R² für eine unter t-Butyl und 1,1-Dimethylpropyl ausgewählte Gruppe steht;
- R₃, R₄ , R₅, R₆, R₇ und R₈ jeweils unabhängig voneinander für ein Wasserstoff- oder Halogenatom oder eine (C₁-C₆) -Alkyl-, (C₁-C₆) -Alkoxy- oder Trifluormethylgruppe stehen; mit der Maßgabe, daß mindestens einer der Substituenten R₃, R₄, R₅, R₆, R₇ und R₈ von Wasserstoff verschieden ist; in Basen- oder Säureadditionssalzform sowie in Hydrat- oder Solvatform.

2. Verbindung nach Anspruch 1, ausgewählt unter 5-(4-Bromphenyl)-4-cyano-1-(2,4-dichlorphenyl)-N-*tert-*butyl-1*H*-pyrazol-3-carboxamid und 5-(4-Bromphenyl)-4-cyano-1-(2,4-dichlorphenyl)-N-(1,1-dimethylpropyl)-1*H*-pyrazol-3-carboxamid;
in Basen- oder Säureadditionssalzform sowie in Hydrat- oder Solvatform.

3. Verfahren zur Herstellung einer Verbindung der Formel (I) nach Anspruch 1, **dadurch gekennzeichnet, daß** man ein funktionelles Derivat von 4-Cyano-1,5-diphenylpyrazol-3-carbonsäure der Formel: 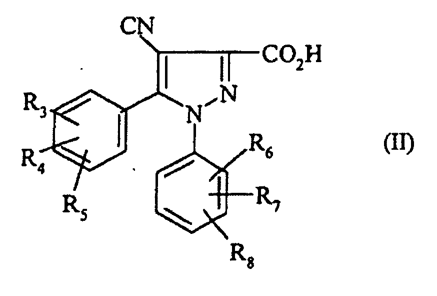 worin R₃, R₄, R₅, R₆, R₇ und R₈ die für (I) in Anspruch 1 angegebene Bedeutung besitzen, mit einem Amin HNR₁R₂ (III), worin R₁ und R₂ die für (I) in Anspruch 1 angegebene Bedeutung besitzen, behandelt.

4. Verbindung der Formel: 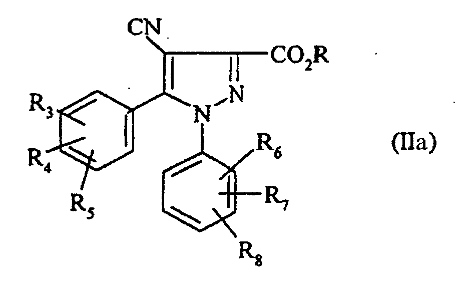 worin:
- R für ein Wasserstoffatom oder eine (C₁-C₄)-Alkylgruppe steht;
- R₃ in 4-Position steht und für ein Chlor- oder Bromatom oder eine Methyl-, Ethyl-, Methoxy- oder Trifluormethylgruppe steht;
- R₆ in 2-Position steht und für ein Chloratom steht;
- R₇ in 4-Position steht und für ein Wasserstoff- oder Chloratom steht;
- R₄, R₅ und R₈ für ein Wasserstoffatom stehen.

5. Arzneimittel, **dadurch gekennzeichnet, daß** es eine Verbindung der Formel (I) nach einem der Ansprüche 1 bis 2 oder ein Additionssalz dieser Verbindung mit einer pharmazeutisch unbedenklichen Säure oder auch ein Hydrat oder ein Solvat enthält.

6. Pharmazeutische Zusammensetzung, **dadurch gekennzeichnet, daß** sie eine Verbindung der Formel (I) nach einem der Ansprüche 1 bis 2 oder ein pharmazeutisch unbedenkliches Additionssalz, ein Hydrat oder ein Solvat dieser Verbindung sowie mindestens einen pharmazeutisch unbedenklichen Hilfsstoff enthält.

7. Verwendung einer Verbindung der Formel (I) nach einem der Ansprüche 1 bis 2 zur Herstellung eines Arzneimittels zur Behandlung von Appetitstörungen, gastrointestinalen Störungen, Entzündungsphänomenen, Erkrankungen des Immunsystems, psychotischen Störungen, Alkoholabhängigkeit und Nikotinabhängigkeit.

8. Verbindung der Formel (I) nach einem der Ansprüche 1 bis 2 zur Verwendung zur Behandlung von Appetitstörungen, gastrointestinalen Störungen, Entzündungsphänomenen, Erkrankungen des Immunsystems, psychotischen Störungen, Alkoholabhängigkeit und Nikotinabhängigkeit.
